# EUROPÄISCHE PATENTANMELDUNG

(11) **EP 0 966 923 A2**
(43) Veröffentlichungstag der Anmeldung: **29.12.1999**
(21) Anmeldenummer: 99110875.4
(22) Anmeldetag: 07.06.1999
(51) Int. Cl.: A61B 17/80

(54) **Plattenanordnung zur Osteosynthese**

(30) Priorität: 26.06.1998 DE 29811479 U
(71) Anmelder: orto MAQUET GmbH & Co. KG, 76437 Rastatt (DE)
(72) Erfinder: Clasbrummel, Bernhard Dr., 44789 Bochum (DE); Gerhardt, Harald Dr., 76131 Karlsruhe (DE)
(74) Vertreter: Schaumburg, Thoenes & Thurn

(57) **Zusammenfassung**

Bei einer Plattenanordnung zur Osteosynthese frakturierter Röhrenknochen mit zwei Plattenteilen (10, 12) die jeweils durch eine Mehrzahl von Befestigungsachrauben (30) mit dein jeweiligen Knochenabschnitt verbindbar und miteinander durch eine Teleskopanordnung verbunden sind, die eine axiale Relativbewegung der Plattenteile (10, 12) ermöglicht, sind die zum Durchtritt der Befestigungsschrauben (30) bestimmten Durchbrechungen (28) in dem Plattenteil (10, 12) von Langlöchern (28) gebildet, deren jeweilige Längsabmessung quer zur Teleskopachse gerichtet ist.

## Beschreibung

Die Erfindung betrifft eine Plattenanordnung zur Osteosynthese frakturierter Röhrenknochen mit zwei Plattenteilen, die jeweils durch eine Mehrzahl von Befestigungsschrauben mit dem jeweiligen Knochenabschnitt verbindbar und miteinander durch eine Teleskopanordnung verbunden sind, die eine axiale Relativbewegung der Plattenteile ermöglicht.

Eine solche Plattenanordnung ist beispielsweise aus der DE-A-41 32 021 bekannt. Die axiale Beweglichkeit der Plattenteile relativ zueinander ermöglicht das Einleiten von kontrollierten Mikrobewegungen in physiologischer Belastungsrichtung, wodurch das Knochenwachstum gefördert werden soll.

Da die Knochen wie beispielsweise Oberschenkelknochen weder alle die gleiche Form und Größe haben noch in sich eine geometrisch gleichförmige Form besitzen, ist die Montage der Plattenteile an den Knochenfragmenten nicht immer problemlos.

Der Erfindung liegt die Aufgabe zugrunde, eine Plattenanordnung der eingangs genannten Art so auszubilden, daß die Montage der Plattenteile an den Knochenabschnitten oder- fragmenten erleichtert wird.

Diese Aufgabe wird erfindungsgemäß dadurch gelöst, daß die zum Durchtritt der Befestigungsschrauben bestimmten Durchbrechungen in den Plattenteilen von Langlöchern gebildet sind, deren jeweilige Längsabmessung quer zur Teleskopachse gerichtet ist.

Die Orientierung der Langlöcher mit ihrer Längsabmessung quer zur Teleskopachse stellt sicher, daß die eingeleiteten Mikrobewegungen im wesentlichen ohne Spiel auf die Knochenfragmente übertragen werden können. Andererseits ermöglichen die Langlöcher das Durchstecken der Schrauben unter einem gewissen Winkel zur Lochachse, so daß man für jede Schraube die hinsichtlich der Befestigung günstigste Stellung wählen kann. Außerdem erlauben die Langlöcher der Plattenanordnung dadurch eine gewisse Kippbewegung um die Teleskopachse und es hat sich überraschenderweise gezeigt, daß auch eine solche kontrollierte Kippbewegung einen fördernden Einfluß auf das Knochenwachstum haben kann.

Vorzugsweise ist die Längsabmessung der Langlöcher relativ zur Dicke des jeweiligen Plattenteiles so gewählt, daß die Schraubenachse relativ zur Langlochachse einen Winkel von bis zu ca. 15° nach beiden Seiten einnehmen kann, so daß der Winkelausschlag zwischen den beiden Extremstellungen ca. 30° betragen kann.

Um eine gute Anlage des Schraubenkopfes an dem jeweiligen Plattenteil zu gewährleisten, auch wenn die Schraube schräg zur Lochachse steht, ist die dem Schraubenschaft nahe Unterseite des Schraubenkopfes vorzugsweise ballig ausgeführt, wobei das Langloch zweckmäßigerweise von einer zur balligen Unterseite des Schraubenkopfes komplementär konkav gekrümmten Auflagefläche umgeben ist. Die Unterseite des Schraubenkopfes bildet somit eine Art Gelenkkugel, die in der als Gelenkpfanne ausgebildeten Auflagefläche gedreht werden kann.

Um dem Operateur die Möglichkeit zu geben, die Plattenteile am Operationsort leichter an die jeweilige Knochenform anpassen zu können, haben die Plattenteile bei einer bevorzugten Ausführungsform der Erfindung in einem mittleren Bereich zwischen je zwei Schraubenlöchern einen verminderten Querschnitt. Dies gibt die Möglichkeit, die Plattenteile in diesem Bereich zu biegen, ohne das die Befestigungsstellen geschwächt werden.

Zweckmäßigerweise ist in der dem Knochen zugewandten Plattenfläche jeweils eine sich parallel zur Teleskopachse erstreckte Aussparung ausgebildet. Dadurch und in Verbindung speziell mit den vorstehend genannten Querschnittsverminderungen ergeben sich auf der zur Anlage an dem Knochen bestimmten Seite des jeweiligen Plattenteiles einzelne Füßchen, über die das Plattenteil an dem Knochen anliegt. Dies ermöglicht eine stabile Mehrpunktauflage des Plattenteiles an dem Knochen.

Um einerseits die Plattenteile verdrehsicher aneinander zu führen, andererseits die Beweglichkeit der Plattenteile relativ zueinander zu verbessern, haben die ineinandergreifenden Abschnitte der Teleskopanordnung bei einer bevorzugten Ausführungsform der Erfindung einen nicht kreisförmigen, vorzugsweise polygonalen Querschnitt, wobei sie über parallel zur Teleskopachse verlaufende Stege aneinander gleiten. Diese Stege können an dem einen oder dem anderen der ineinandergreifenden Teleskopabschnitte ausgebildet sein. Dadurch ist einerseits eine sichere Führung und andererseits eine verminderte Reibung der Teile aneinander gewährleistet. Weitere Merkmale und Vorteile der Erfindung ergeben sich aus der folgenden Beschreibung, welche in Verbindung mit dem beigefügten Zeichnungen die Erfindung an Hand von Ausführungsbeispielen erläutert. Es zeigen:
- Fig. 1: eine Seitenansicht einer erfindungsgemäßen Plattenanordnung,
- Fig. 2: eine Draufsicht auf die in Fig. 1 dargestellte Plattenanordnung,
- Fig. 3: einen Schnitt durch die Plattenanordnung entlang Linie II-II in Fig. 1,
- Fig. 4: eine Seitenansicht einer abgewandelten Ausführungsform eines Plattenteiles,
- Fig. 5: eine Draufsicht auf das in Fig. 4 dargestellte Plattenteil,
- Fig. 6: eine Endansicht auf das in den Fig. 4 und 5 dargestellte Plattenteil von links,
- Fig. 7: einen Schnitt durch das Plattenteil entlang Linie VII-VII in Fig. 4,
- Fig. 8: einen Schnitt durch das Plattenteil entlang Linie VIII-VIII in Fig. 5,
- Fig. 9: eine schematische vergrößerte Draufsicht auf den Befestigungsabschnitt eines Plattenteiles mit einem Langloch gemäß einer weiteren Ausführungsform der Erfindung,
- Fig. 10: einen schematischen Querschnitt entlang Linie X-X in Fig. 9 und
- Fig. 11 bis 14: jeweils den Fig. 9 und 10 entsprechende Ansichten zweier weiterer Ausführungsformen der Erfindung.

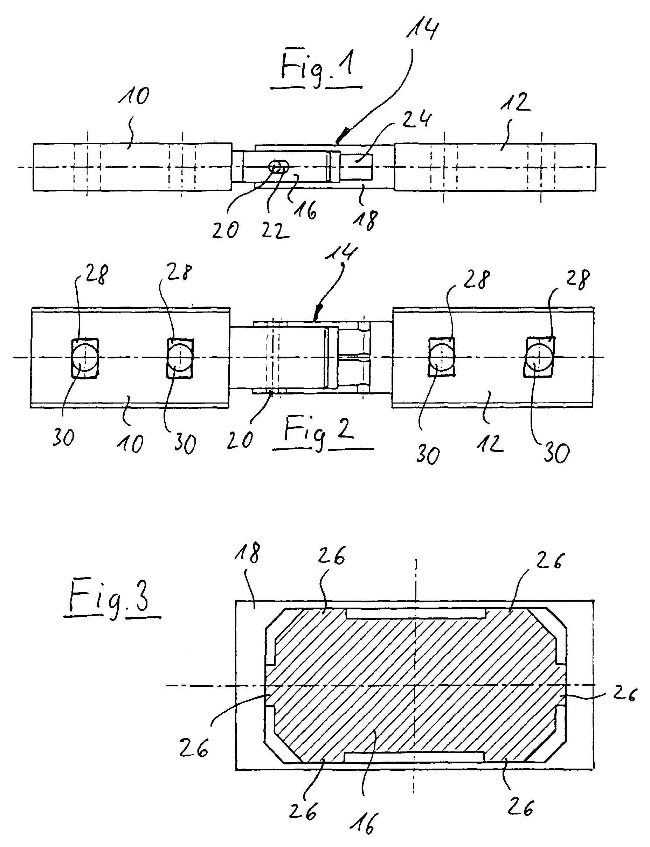

Die in den Fig. 1 und 2 dargestellte Plattenanordnung zur Osteosynthese frakturierter Röhrenknochen umfaßt ein erstes Plattenteil 10 und ein zweites Plattenteil 12, die durch einen Teleskopmechanismus 14 miteinander verbunden sind. Dieser umfaßt ein mit dem ersten Plattenteil verbundenes Zapfenelement 16, das in ein mit dem zweiten Plattenteil verbundenes Hülsenelement 18 eingreift. In der zusammengesteckten Stellung werden die beiden Plattenteile 10 und 12 durch einen Bolzen 20 gesichert, der das Zapfenelement 16 durchsetzt und in dem Hülsen- oder Führungsteil 18 in Langlöchern 22 gelagert ist, deren Längsrichtung parallel zur Teleskopachse 24 verläuft. Die Langlöcher 22 ermöglichen somit eine begrenzte Bewegung der Plattenteile 10 und 12 in axialer Richtung relativ zueinander.

Fig. 3 zeigt den Querschnitt der ineinandergreifenden Teleskopteile 16 und 18. Man erkennt, daß das Zapfelement 16 nicht vollflächig, sondern nur über Stege 26 an den Wänden des einen polygonalen Querschnitt aufweisenden Führungsteils 18 anliegt.

In jedem Plattenteil 10 und 12 sind gemäß Fig. 2 langlochartige Durchbrechungen 28 zur Aufnahme von Befestigungsschrauben ausgebildet. Die Langlöcher 28 sind dabei mit ihrer Längsrichtung quer zur Teleskopachse 24 gerichtet, so daß die jeweils durch einen Kreis angedeuteten Befestigungsschraube 30 sich zwar quer zur Teleskopachse 24 nicht aber in Richtung derselben bewegen können.

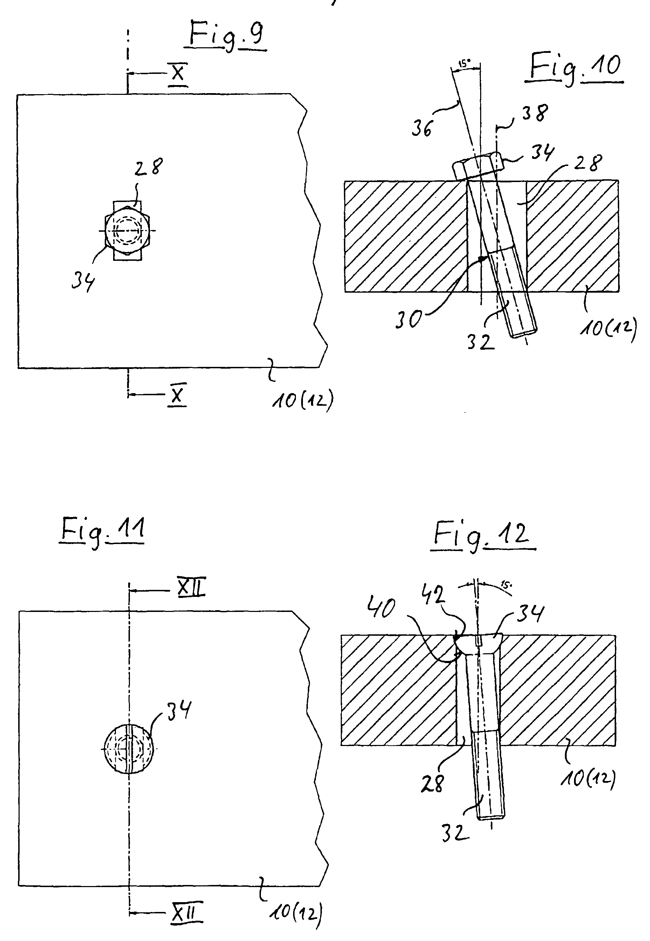

Die Fig. 9 bis 14 zeigen drei Ausführungsvarianten für die Anordnung der Befestigungsschrauben in den Durchbrechungen 28, wobei jeweils gleiche Teile mit gleichen Bezugszeichen versehen sind.

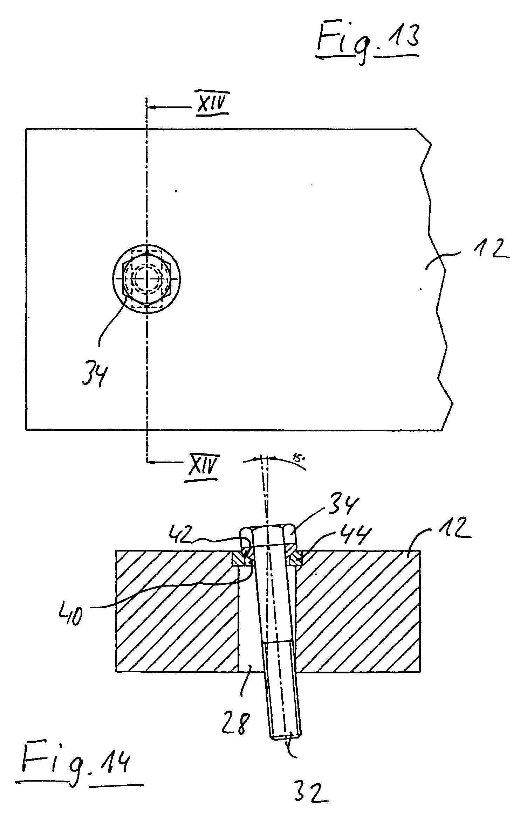

In Fig. 10 erkennt man, daß die Schraube 30 mit einem Schaftteil 32 und einem Schraubenkopf 34 in dem Langloch 28 in der Längsrichtung desselben hin und her pendeln kann und zwar so, daß die Schraubenachse 36 mit der Lochachse 38 einen Winkel von ± 15° bilden kann. Dies ermöglicht es einerseits, die günstigste Stellung für das Eintreiben der Schrauben in den Knochen zu wählen. Andererseits erhält dadurch das jeweilige Plattenteil eine gewisse Beweglichkeit gegenüber dem Knochen quer zur Teleskopachse.

Bei dem in den Fig. 11 und 12 dargestellten Ausführungsbeispiel hat der Schraubenkopf 34 eine kugelkalottenförmige ballige Unterseite 40, mit der er in einer komplementär gekrümmten Auflagefläche 42 wie in einer Gelenkpfanne liegt.

Bei der in den Fig. 13 und 14 dargestellten Ausführungsform hat der Schraubenkopf ebenfalls eine ballig gekrümmte Unterseite 40, wobei die Auflagefläche 42 an einem Ring 44 ausgebildet ist, der in die Durchbrechung 28 eingesetzt ist.

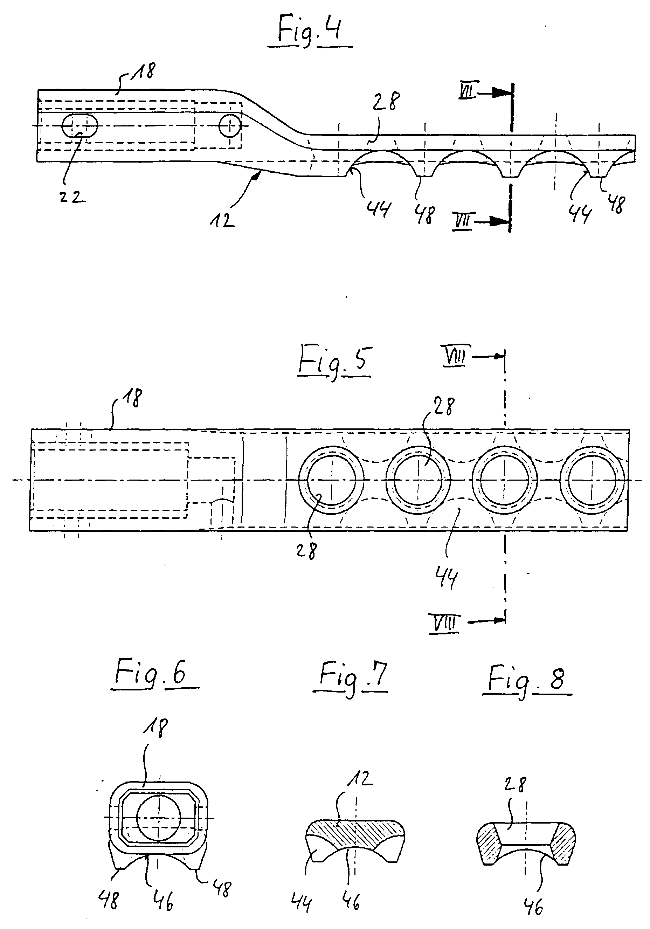

Die Fig. 4 bis 8 zeigen eine spezielle Ausführungsform des zweiten Plattenteiles 12. Gleiche Teile sind mit wiederum mit gleichen Bezugszeichen versehen. Ein wesentliches Merkmal dieser Ausführungsform besteht darin, daß das Plattenteil zwischen je zwei Schraubenlöchern 28 an seiner Unterseite eine kugelkalottenförmige Aussparung 44 hat, durch die zwischen je zwei Schraubenlöchern der Plattenquerschnitt vermindert wird. Dies gibt dem Chirurgen die Möglichkeit, an Ort und Stelle das Plattenteil zu biegen, um es an einen Knochen besser anpassen zu können. Ferner ist an der Unterseite des Plattenteiles, d.h. der dem Knochen zugewandten Seite desselben eine teilzylindrische Aussparung 46 ausgebildet, deren Achse parallel zur Teleskopachse verläuft. In Kombination mit den Ausparungen 44 entstehen dadurch seitlich der Schraubenlöcher 28 jeweils Füßchen 48, mit denen das Plattenteil 12 auf dem Knochen an mehreren Punkten aufliegt.

## Patentansprüche

1. Plattenanordnung zur Osteosynthese frakturierter Röhrenknochen mit zwei Plattenteilen (10, 12,) die jeweils durch eine Mehrzahl von Befestigungsschrauben (30) mit dem jeweiligen Knochenabschnitt verbindbar und miteinander durch eine Teleskopanordnung (16, 18) verbunden sind, die eine axiale Relativbewegung der Plattenteile (10, 12) ermöglicht, dadurch **gekennzeichnet,** daß die zum Durchtritt der Befestigungsschrauben (30) bestimmten Durchbrechungen (28) in dem Plattenteil (10, 12) von Langlöchern gebildet sind, deren jeweilige Längsabmessung quer zur Teleskopachse (24) gerichtet ist.

2. Plattenanordnung nach Anspruch 1, dadurch **gekennzeichnet,** daß die Längsabmessung der Langlöcher (28) relativ zur Dicke des jeweiligen Plattenteiles (10, 12) so gewählt ist, daß die Schraubenachse (36) relativ zur Langlochachse (38) einen Winkel bis zu ca. ± 15° einnehmen kann.

3. Plattenanordnung nach Anspruch 1 oder 2, dadurch **gekennzeichnet,** daß die dem Schraubenschaft (32) nahe Unterseite (40) des Schraubenkopfes (34) ballig ausgeführt ist.

4. Plattenanordnung nach Anspruch 3, dadurch **gekennzeichnet,** daß das Langloch (28) von einer zur balligen Unterseite (40) des Schraubenkopfes (34) komplementär konkav gekrümmten Auflagefläche (42) umgeben ist.

5. Plattenanordnung nach einem der Ansprüche 1 bis 4, dadurch **gekennzeichnet,** daß die Plattenteile (10, 12) in einem mittleren Bereich zwischen je zwei Schraubenlöchern (28) einen verminderten Querschnitt haben.

6. Plattenanordnung nach einem der Ansprüche 1 bis 5, dadurch **gekennzeichnet,** daß in der dem Knochen zugewandten Plattenfläche der Plattenteile (10, 12) eine sich parallel zur Teleskopachse (24) erstreckende Aussparung ausgebildet ist.

7. Plattenanordnung nach einem der Ansprüche 1 bis 6, dadurch **gekennzeichnet,** daß die ineinandergreifenden Abschnitte der Teleskopanordnung (16, 18) einen nicht kreisförmigen, vorzugsweise polygonalen Querschnitt haben und daß sie über parallel zur Telekopachse (24) verlaufende Stege (26) aneinander gleiten.
